(19) Europäisches Patentamt
European Patent Office
Office européen des brevets

(11) Publication number: 0 114 658
B1

(12) **EUROPEAN PATENT SPECIFICATION**

(45) Date of publication of patent specification: **27.07.88**

(21) Application number: **84100508.5**

(22) Date of filing: **18.01.84**

(51) Int. Cl.[4]: **C 07 D 211/48**, C 07 D 211/58, C 07 D 401/14, C 07 D 401/06, C 07 D 401/12, C 07 F 9/65, C 08 K 5/34, C 07 D 221/20

(54) **Alkyl-substituted 4-methyl-piperidine derivatives and use thereof as stabilizers.**

(30) Priority: **19.01.83 IT 1917283**

(43) Date of publication of application: **01.08.84 Bulletin 84/31**

(45) Publication of the grant of the patent: **27.07.88 Bulletin 88/30**

(84) Designated Contracting States: **BE CH DE FR GB LI NL**

(56) References cited:
**EP-A-0 003 542**
**EP-A-0 060 559**
**BE-A- 891 835**
**DE-A-2 851 898**
**GB-A-2 074 564**

(73) Proprietor: **Montedison S.p.A.**
**31, Foro Buonaparte**
**I-20121 Milan (IT)**

(72) Inventor: **di Battista, Piero, Dr.**
**52/2, via Matteotti**
**Peschiere Borromeo (Milano) (IT)**

(74) Representative: **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40 (DE)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 114 658 B1

**Description**

This invention relates to new derivatives of alkyl-substituted 4-methyl-piperidine, the preparation thereof and to polymeric compositions stabilized with such derivatives.

As it is known, polymeric substances are generally subject to degradation, such as discoloration and embrittlement, caused by exposure to light, especially to ultraviolet light, and by the action of oxygen and heat.

In order to prevent or delay such degradation, it is a common technique to add to the polymeric materials various types of compounds opposing such effect, generally referred to as stabilizers.

A particular type of stabilizers which have been very successful are the sterically hindered amines (HALS) and in particular the derivatives of alkyl-substituted piperidine.

Derivatives of alkyl-substituted-4-oxy-4-methyl-piperidine are also known. Thus, e.g., from EP—A—60,559 there are known polymeric products containing, as a repeating unit, alkyl-substituted 4-oxy-4-methylene piperidines, and from BE—A—891, 835 there are known alkyl-substituted 4-oxy-4-amino-methylpiperidines and derivatives thereof containing an alkyl-carbonyl radical or the triazine radical, bound to the amine nitrogen.

Although all these known compounds, containing the 4-oxy-methyl-piperidine radical, are excellent stabilizers for polymeric substances generally subject to deterioration, they cannot be utilized, with the same favourable results, in all the polymeric substances and in all applications. In fact, the known stabilizers exhibit the drawback of not possessing all the parameters and characteristics required for all technical applications such as low volatility, resistance to migration, thermal stability and insolubility in water.

One object of the present invention is to improve the properties of the known stabilizers containing the alkyl-substituted 4-oxy-4-methyl-piperidine radical.

Accordingly, the present invention provides derivatives of alkyl-substituted 4-methyl-piperidine derivatives of formula (I):

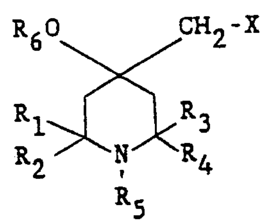

(I)

wherein:

$R_1$, $R_2$, $R_3$ and $R_4$, which may be the same or different, represent an alkyl radical having from 1 to 4 carbon atoms, or $R_1$ and $R_2$ and/or $R_3$ and $R_4$ together form an alkylene group having from 2 to 11 carbon atoms;

$R_5$ is hydrogen, an alkyl radical containing from 1 to 4 carbon atoms, a hydroxyalkyl or alkoxy radical containing from 1 to 6 carbon atoms, an aryl or aryl-alkyl radical containing from 6 to 18 carbon atoms, an alkenyl radical having from 2 to 6 carbon atoms, or the group:

$$-CH_2-\underset{\displaystyle OR_8}{\underset{|}{CH}}-R_7 \qquad a)$$

wherein

$R_7$ is hydrogen, an alkyl radical containing from 1 to 4 carbon atoms or an alkoxy radical containing from 1 to 6 carbon atoms, and

$R_8$ is hydrogen, an alkyl radical containing from 1 to 4 carbon atoms, or the group:

$$-\overset{\displaystyle O}{\overset{\|}{C}}-R_9 \qquad b)$$

wherein

$R_9$ is hydrogen, an alkyl radical containing from 1 to 18 carbon atoms, a phenyl or benzyl radical or the group:

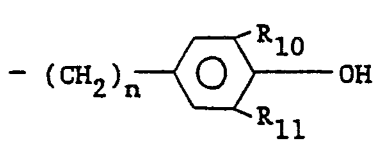

c)

wherein

*n* is an integer from 1 to 10, and $R_{10}$ and $R_{11}$ is hydrogen or an alkyl radical containing from 1 to 4 carbon atoms;

$R_6$ is hydrogen, an alkyl radical, an alkyl-carbonyl radical, in which the alkyl group contains from 1 to 22 carbon atoms, a phenyl-alkyl-carbonyl radical, a cyclo-alkyl-carbonyl radical or a phenyl-carbonyl radical, in which the phenyl or cycloalkyl nucleus is optionally substituted by alkyl groups containing from 1 to 3 carbon atoms; and

X may be:

A) —$NR_{12}$, $R_{13}$, in which $R_{12}$ and $R_{13}$, which may be the same or different from each other, represent:

an alkyl radical containing from 1 to 10 carbon atoms;

an alkenyl or alkynyl radical containing from 2 to 10 carbon atoms;

benzyl, phenyl or the corresponding alkyl-substituted radicals;

the radical:

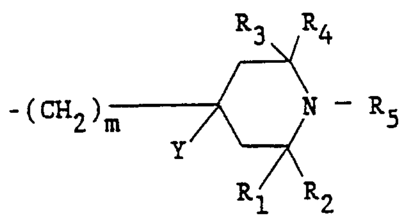

d)

wherein:

$R_1$, $R_2$, $R_3$, $R_4$ and $R_5$ have the meaning indicated hereinbefore, *m* is 0 or an integer from 1 to 3 and Y is hydrogen, a hydroxyl group, a hydroxyalkyl-carbonyl radical, a hydroxy-phenyl-carbonyl-radical, a hydroxy-cycloalkyl-carbonyl radical, in which the phenyl or cycloalkyl group may be alkyl-substituted;

the radical:

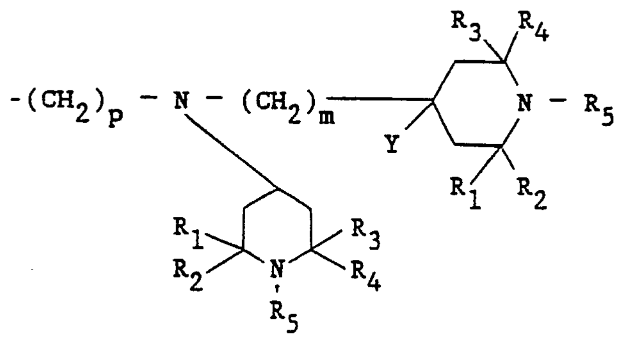

e)

wherein:

$R_1$, $R_2$, $R_3$, $R_4$, $R_5$, m and Y have the same meaning explained hereinbefore and p is an integer ranging from 1 to 12;

the radical

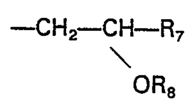

f)

wherein:

$R_7$ and $R_8$ have the meanings indicated hereinabove;

the triazine radical

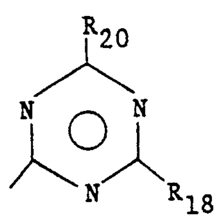

g)

wherein

$R_{20}$ and $R_{18}$, which may be the same or different, each represent an alkyl radical containing from 1 to 18 carbon atoms, an alkoxy radical containing from 1 to 18 carbon atoms, or the radical:

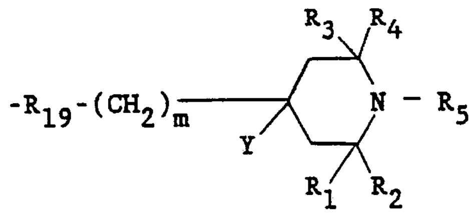

wherein:

$R_1$, $R_2$, $R_3$, $R_4$, $R_5$, m and Y have the meanings as indicated hereinbefore and $R_{19}$ may be oxygen or —N—$R_{12}$; or

$R_{12}$ and $R_{13}$ form, along with the nitrogen atom they are linked to, an unsubstituted or alkyl-substituted morpholino or piperidino group; or

$R_{12}$ and $R_{13}$ together form the group:

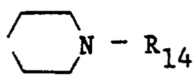

h)

unsubstituted or alkyl-substituted in the nucleus, in which $R_{14}$ is hydrogen, an alkyl radical containing from 1 to 6 carbon atoms, or the radical:

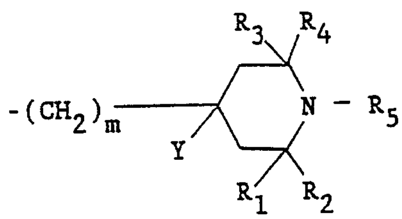

i)

wherein

m, $R_1$, $R_2$, $R_3$, $R_4$, $R_5$ and Y have the meanings indicated hereinbefore; or

$R_{12}$ or $R_{13}$ may be hydrogen, with the proviso that neither is hydrogen when radical g) is present;

B)

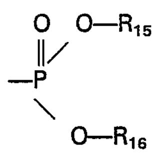

in which $R_{15}$ and $R_{16}$, which may be the same or different from each other, each represent hydrogen, an alkyl radical containing from 1 to 6 carbon atoms, or the group:

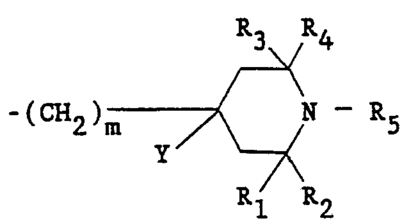

in which $R_1$, $R_2$, $R_3$, $R_4$, $R_5$, Y and m have the meaning indicated hereinbefore; or

C) —O—$R_{17}$, in which $R_{17}$ may be:

hydrogen;

an alkyl, alkenyl or alkynyl radical, each containing from 1 to 18 carbon atoms;

a benzyl or phenyl radical, optionally substituted in the nucleus;

the radical:

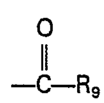

b)

in which $R_9$ has the meaning indicated hereinabove; or

the triazine radical

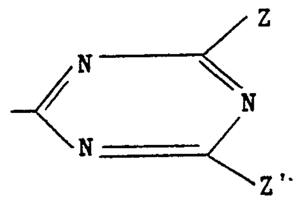

n)

in which: Z and Z′, which may be the same or different, each represent hydrogen, an alkyl radical containing from 1 to 18 carbon atoms, an alkoxy or alkylamino group in which the alkyl radical contains from 1 to 18 carbon atoms, or the radical:

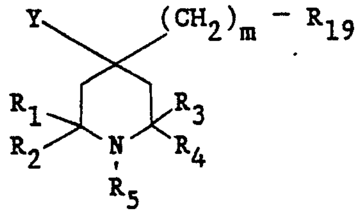

in which $R_1$, $R_2$, $R_3$, $R_4$, $R_5$, $R_{19}$, Y and m have the meanings indicated hereinbefore.

All the alkyl, alkenyl or alkynyl groups containing 3 carbon atoms or more may have a linear or branched chain.

In the above formulae alkyl residues with 1 to 18 carbon atoms (straight or branched) are e.g. methyl, ethyl, n- and i-propyl, n-butyl, i-butyl, sec.-butyl, tert.-butyl, n- and i-pentyl, n- and i-hexyl, n- and i-heptyl, n- and i-octyl, nonyl, decyl, dodecyl, tetradecyl, hexadecyl, and octadecyl; if the chain of the alkyl group is shorter, e.g. only 1 to 6 carbon atoms, then the examples given above up to the corresponding upper limit are applicable (in this case up to 6 carbon atoms, i.e. n- and i-hexyl); if the group contains more than 18 carbon atoms, e.g. up to 22 C-atoms, this group may also be eicosyl etc. (preferably, however, 1 to 18 C-atoms); alkenyl groups are e.g. vinyl, allyl, propenyl, the butenyl residues, pentenyl and unsaturated residues corresponding to the above mentioned alkyl residues, for alkynyl groups the same is applicable as above mentioned for the alkenyl groups (ethynyl, propynyl, etc. up to octadecynyl).

If the alkyl groups are not defined by their chain length they contain 1 to 8, preferably 1 to 6 and most preferably 1 to 4 carbon atoms.

The above given definition for alkyl is also valid for more complex residues like alkylamino or alkoxy.

Arylalkyl with 6 to 18 carbon atoms can be a group $C_6H_5$-alkyl $C_1$ to $C_{12}$ or an alkyl substituted aryl connected with a corresponding alkyl group.

Cycloalkyl with 3 to 12 carbon atoms (preferably 3 to 8 carbon atoms) are cyclopropyl, cyclobutyl, cyclopentyl, cyclohexyl, cycloheptyl, cyclooctyl, and cyclododecyl which may be substituted by methyl or ethyl (up to the upper limit of 12 carbon atoms).

The present invention also provides compositions based on synthetic polymers, stabilized as to oxidation and to ageing, containing, as a stabilizer, an alkyl-substituted-4-methyl-piperidine derivative having the formula (I) in an amount sufficient to prevent any degradation of the polymer.

In preferred embodiments of the present invention the alkyl-substituted 4-methyl-piperidine derivatives of formula (I) are those in which:

$R_1$, $R_2$, $R_3$ and $R_4$ are methyl or ethyl radicals; and/or

$R_5$ is hydrogen, a methyl or allyl radical; and/or

$R_6$ is hydrogen, or a methyl or ethyl radical; and/or

X is selected from

A) —$NR_{12}R_{13}$ in which

$R_{12}$ and $R_{13}$ both represent an alkyl radical containing from 1 to 10 carbon atoms; or

one of $R_{12}$ and $R_{13}$ represents the radical d) as defined above (M = O) and the other one is an alkyl radical containing from 1 to 10 carbon atoms; or

at least one of $R_{12}$ and $R_{13}$ is the radical e) as defined above; or

$R_{12}$ and $R_{13}$ together form the alkyl-substituted group h) as defined above; or

at least one of $R_{12}$ and $R_{13}$ represents the radical g) as defined above wherein $R_{18}$ and $R_{20}$ both are alkoxy radicals containing from 1 to 18 carbon atoms;

B)

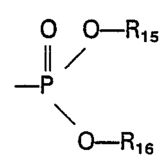

in which $R_{15}$ and $R_{16}$ are as defined above;

C) —O—$R_{17}$ in which $R_{17}$ may be

an alkyl radical having from 1 to 18 carbon atoms; or

the radical n) as defined above wherein Z and Z′ both are either alkoxy groups in which the alkyl radical contains from 1 to 18 carbon atoms or represent the radical

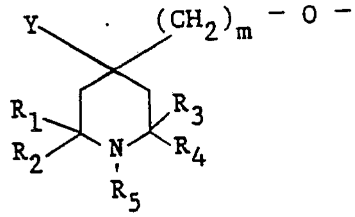

wherein $R_1$, $R_2$, $R_3$, $R_4$, $R_5$, Y and m are as defined above.

Examples of alkyl-substituted 4-methyl-piperidine-derivatives having formula (I), according to the present invention, are:

A. among the compounds in which X = $NR_{12}R_{13}$:

I

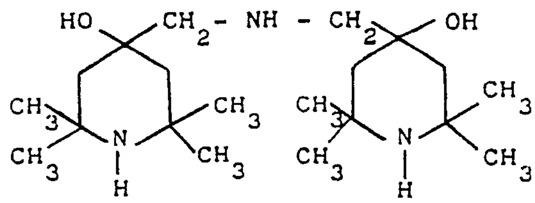

II

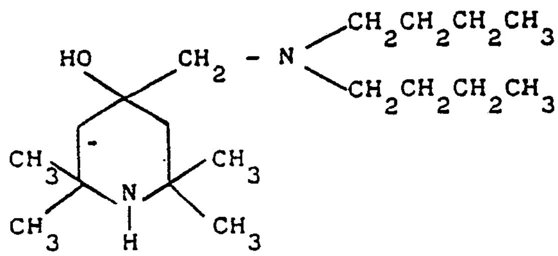

III

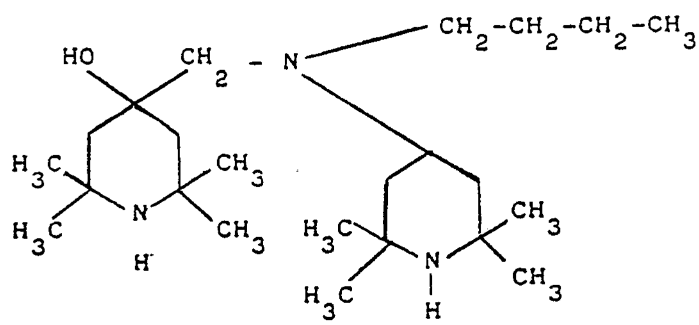

IV

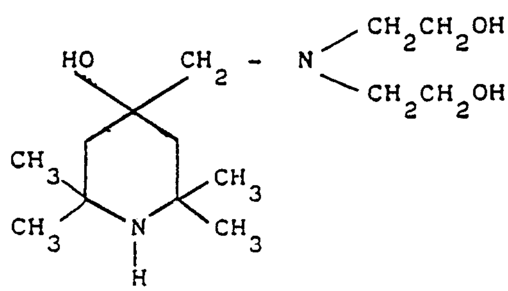

V

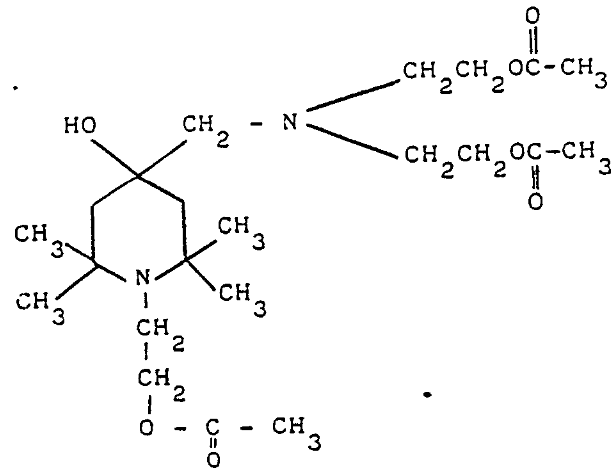

VI

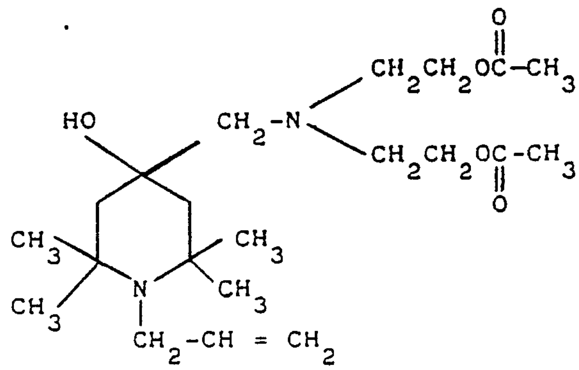

VII

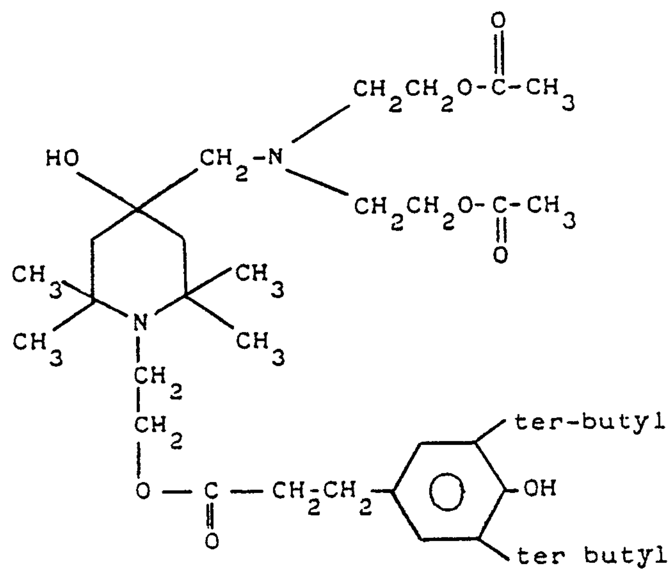

VIII

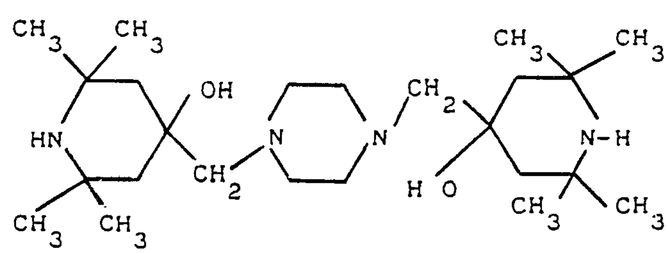

IX

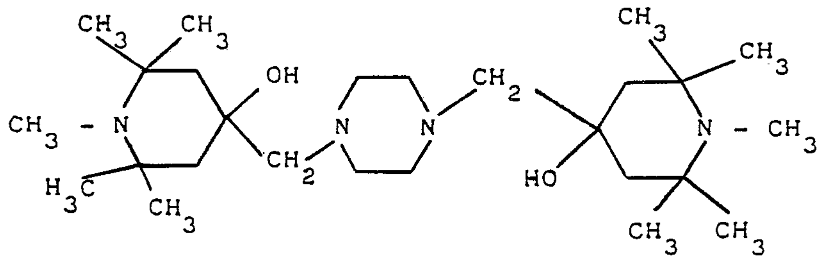

X

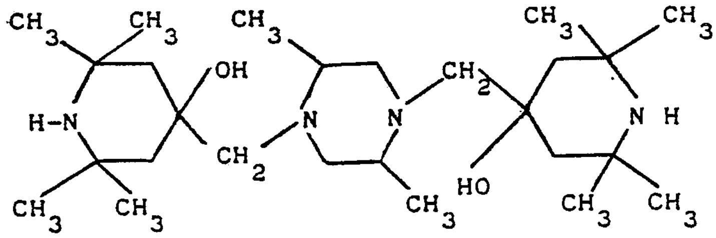

XI

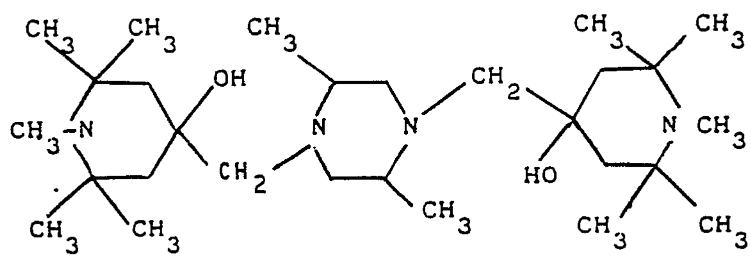

XII

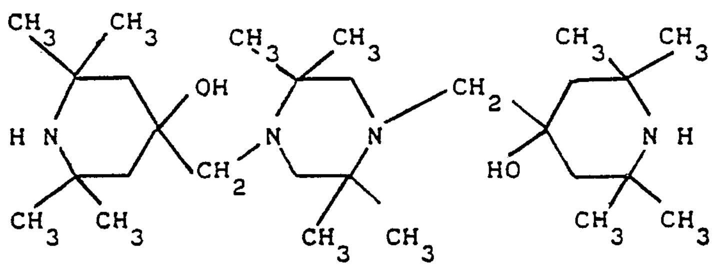

XIII

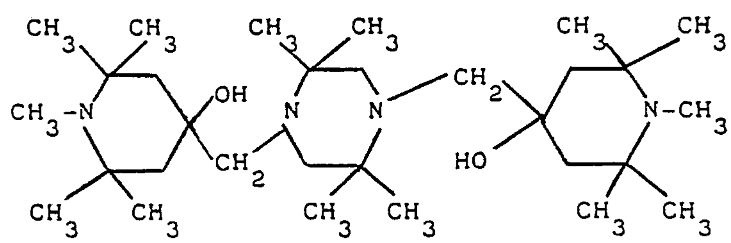

XIV

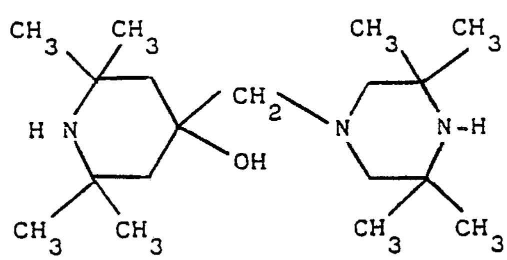

XV

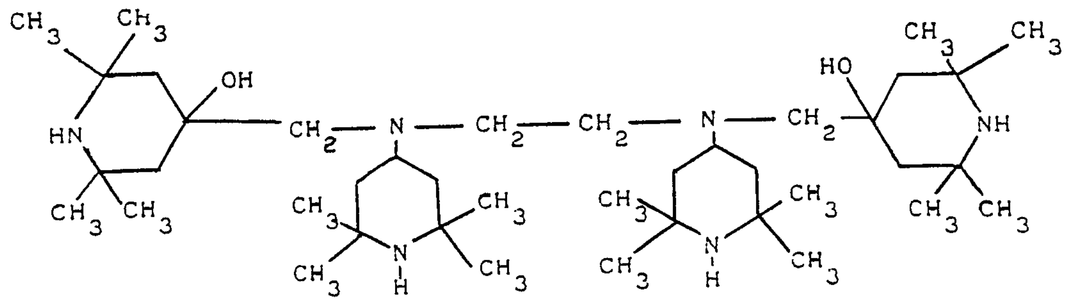

XVI

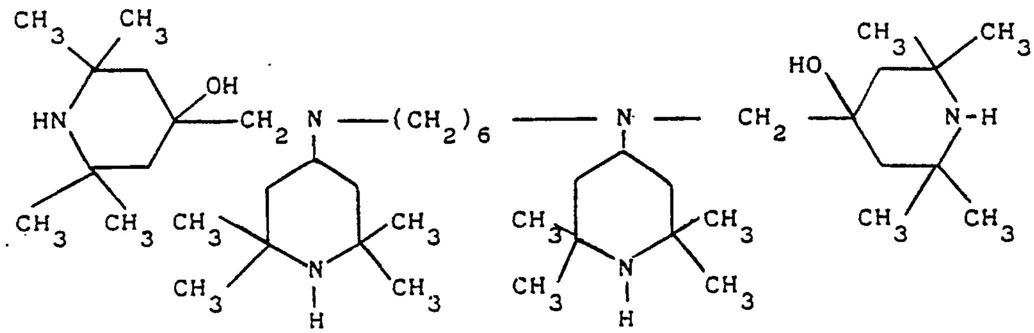

XVII

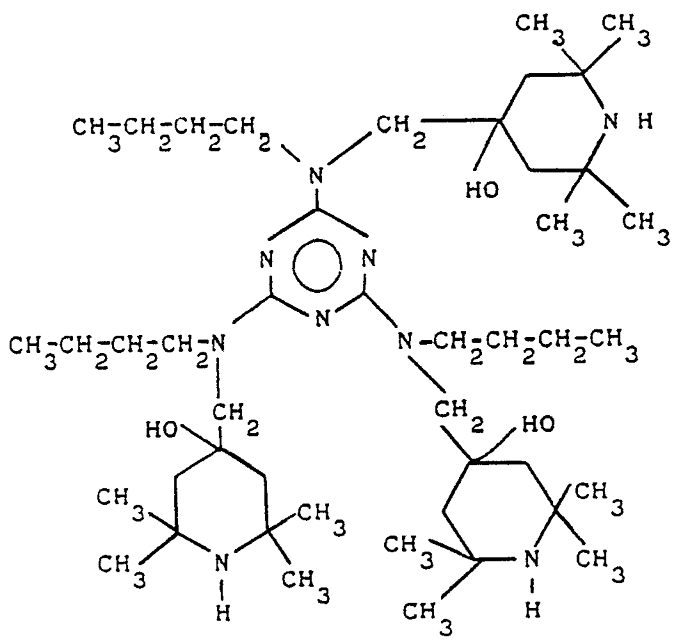

XVIII

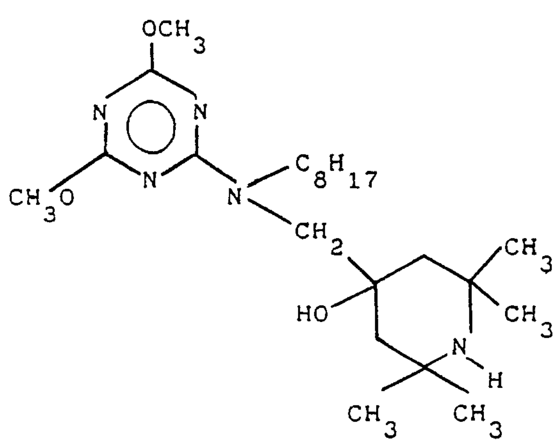

XIX

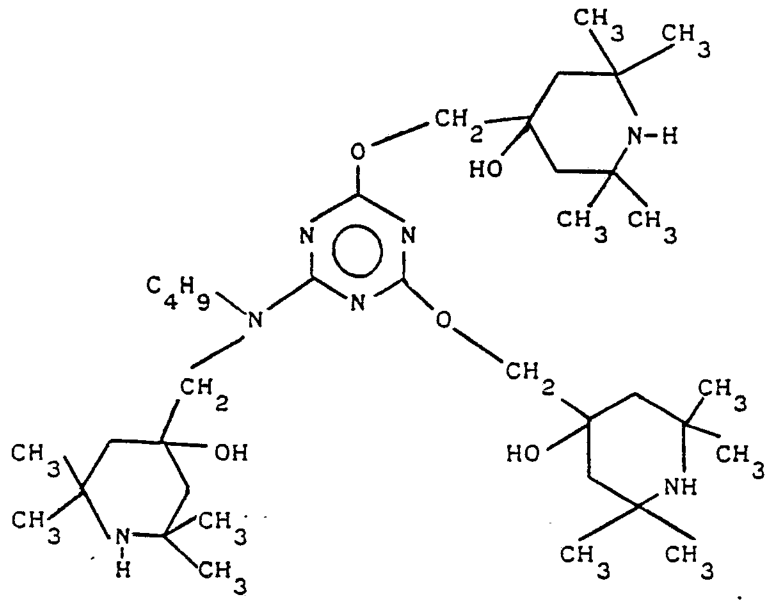

B. Among the compounds wherein X = - 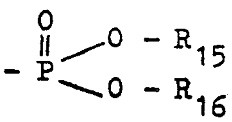 ;

XX

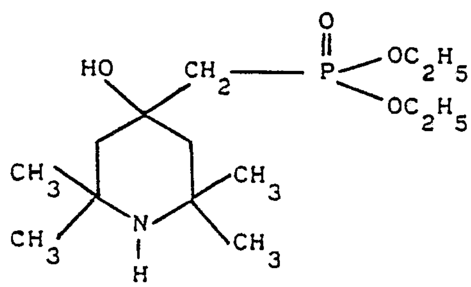

XXI

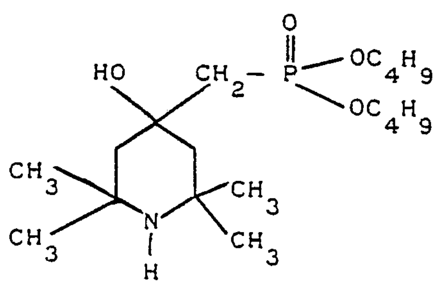

XXII

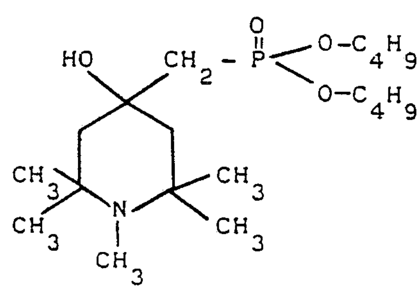

XXIII 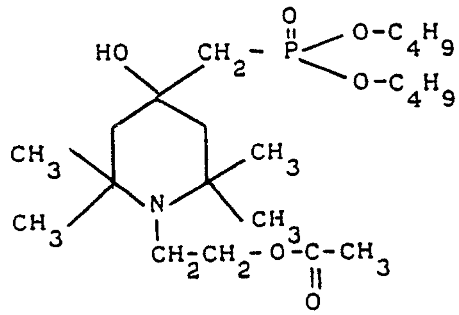

C. Among the compounds wherein $X = O - R_{17}$ :

XXIV 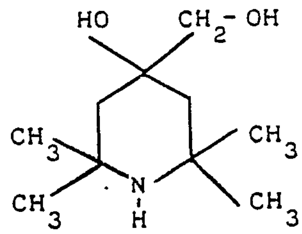

XXV 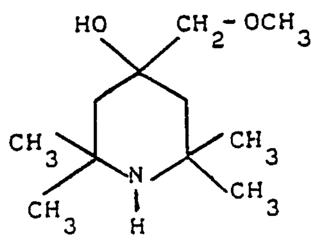

XXVI 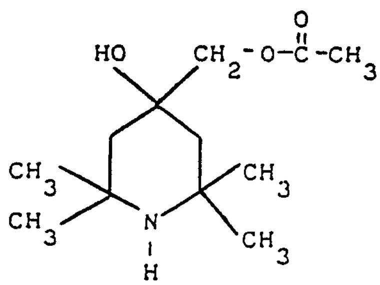

XXVII 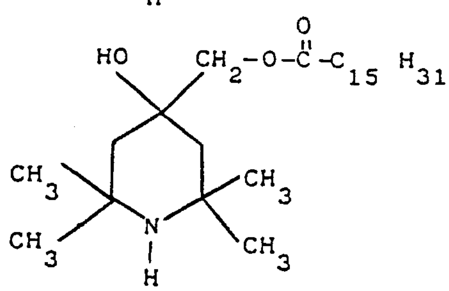

XXVIII 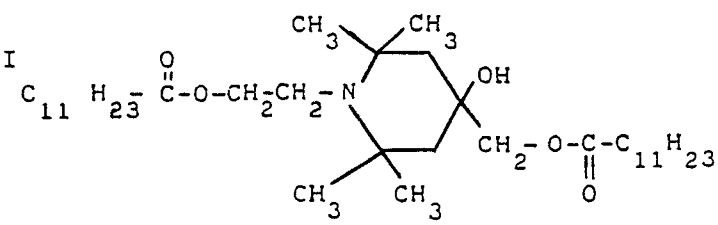

XXIX

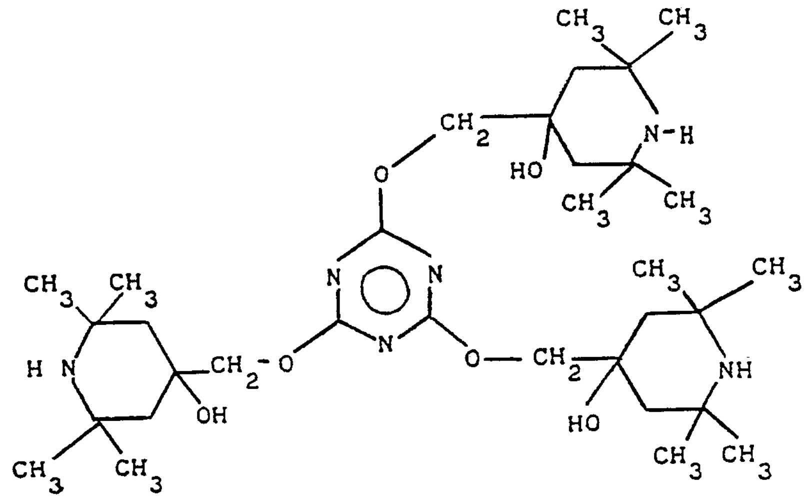

The alkyl-substituted 4-methyl-piperidine derivatives of formula (I) may be synthesized by starting from an alkyl-piperidyl-4-spiro-oxirane or a derivative thereof. In particular, the compounds having formula (I) in which "X" is $—NR_{12}R_{13}$ are synthesized by reacting, in an equimolar amount, preferably in the presence of an inert organic solvent and at a temperature ranging from room temperature to 200°C, an alkyl-piperidyl-4-spiro-oxirane or a derivative thereof with a primary or secondary amine. Suitable organic solvents may be e.g. alcohols, ethers and halogen hydrocarbons.

Methyl alcohol is preferred. Also water can be employed as a solvent.

The compounds having formula (I) in which:

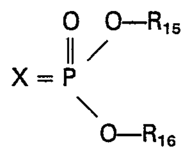

may be synthesized by reacting, in equimolar amounts, preferably in the presence of an inert solvent and at a temperature ranging from room temperature to 200°C, an alkyl-piperidyl-4-spiro-oxirane or a derivative thereof with a phosphite. Suitable solvents may be e.g. water, alcohols, ethers and halogenated hydrocarbons. It is furthermore preferred to carry out the reaction in the presence of organic bases such as pyridine, triethyl-amine, alcoholates of alkali metals or of alkaline-earth metals, hydrides or amides of alkali or alkaline-earth metals, or generally substances containing quaternary ammonium halides. The amount of these organic bases in the reaction medium ranges from small catalytic amounts, such as 0.001%, up to above the stoichiometric amounts.

The compounds of formula (I) in which X is $—O—R_{17}$ may be synthesized by reacting, in equimolar amounts, preferably in the presence of an inert organic solvent and at a temperature ranging from room temperature to 200°C, an alkyl-piperidyl-4-spiro-oxirane or a derivative thereof with an alcoholate of an alkali or alkaline-earth metal. Suitable inert organic solvents may be e.g. ethers, hydrocarbons, halogenated hydrocarbons and aromatic hydrocarbons such as xylene, toluene. At the end of the reaction, a washing step with water is carried out and the reaction product is recovered by evaporating the organic solvent.

The compounds of formula (I) in which X is $O—R_{17}$ and $R_{17}$ is the radical

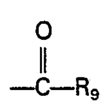

or the triazine radical, may be prepared by reacting the compound of formula XXIV with an halogen-derivative containing an acyl- or triazinyl radical, in the presence of a base such as an hydroxide or carbonate of an alkali metal and optionally of an inert solvent, at a temperature from room temperature to 200°C.

The alkyl-substituted 4-methyl-piperidine derivatives of formula (I) of the present invention, besides possessing the well-known photostabilizing properties of the HALS, exhibit a surprisingly improved stabilizing effect with respect to thermo-oxidation, accompanied by a substantial water-insolubility, non-volatily and a high stability to heat.

Examples of materials which can be stabilized by the derivatives of the invention are mainly the synthetic organic polymeric substances, including:

polyolefins, such as homopolymers of olefins, e.g. high density and low density polyethylene, polypropylene, polystyrene, polybutene and polyisoprene; the copolymers of olefins with other ethylenically unsaturated monomers such as ethylene-propylene copolymers, ethylene-butene copolymers, styrene-butadiene and styrene-acrylonitrile copolymers; the terpolymers of ethylene, propylene and a diene such as acrylonitrile-styrene-butadiene; and the mixtures of the above-mentioned polymers and copolymers;

vinyl polymers containing halogen, such as polyvinyl chloride and polyvinylidene chloride, polyvinyl fluoride, chlorinated rubbers, the copolymers of vinyl chloride and of vinylidene chloride with each other or each with vinyl acetate or other ethylenically unsaturated monomers;

polymers derived from α,β unsaturated acids or derivatives thereof, such as polyacrylates, polymethacrylates, polyacrylamides, polyacrylonitrile and also the copolymers thereof;

polyacetals such as polyoxymethylene and polyoxyethylene;

polyesters such as polyethylene-terephthalates;

polyamides such as nylon 6, nylon 6—6 and nylon 6—10; and the corresponding co-polyamides;

polyurethanes and polyureas;

polymers derived from unsaturated alcohols and from unsaturated amines;

polycarbonates;

monomers and copolymers derived from epoxides such as polyethylene-oxides;

thermoplastic elastomers;

natural and synthetic rubbers, etc.

Such synthetic polymers may be employed either as powder or granules, or as shaped articles, e.g. fibres, films, sheets and foils; or as latices and foams.

Of the synthetic polymers cited hereinabove, the ones which are more suited to be stabilized, according to the present invention, are the polyolefines derived from monomers having the general formula: $R—CH=CH_2$, wherein R is an alkyl or aryl group, or a hydrogen atom.

Among the polyolefins, polypropylene prevailingly consisting of isotactic macromolecules and obtained by stereospecific polymerization of propylene is preferably stabilized.

The amount of alkyl-substituted 4-methyl-piperidine derivative having formula (I) to be added to the substance to be stabilized, according to this invention, is not critical and may vary over a wide range as a function of the type, properties and particular uses of the substance. Generally, said stabilizers can be added in amounts ranging from 0.01 to 5.0% by weight.

Practically, however, the amount varies as a function of the type of substance to be stabilized. Thus, for example, in the case of the polyolefines, such amount varies from 0. 01 to 2% by weight; for the halogen-containing vinyl polymers, such amount may range from 0.01 to 1% by weight; and for the polyurethanes, the polyamides and the polymers derived from α,β-unsaturated acids, the amount may vary from 0.01 to 5% by weight. The derivatives of the alkyl-substituted 4-methyl-piperidines having formula (I) may be employed either alone or in admixture with other known additives such as antioxidants, UV-ray absorbers, pigments, fillers, basic nitrogen containing polycondensates and stabilizers.

Examples of such additives are oxo-benzo-triazoles, oxo-benzo-phenones, Ni-stabilizers, metal soaps, phenolic antioxidants, phosphites, phosphinites, thioesters, hydroquinone derivatives, triazinic compounds, acylamino-phenols, benzyl-phosphonates, sterically hindered phenols such as 4,4'-bis-butylidene-bis-(2,6-di-ter.butyl)-phenol and triazino-phenol compounds.

Such additives can be utilized along with the compounds having formula (I) in a weight ratio in the range of from 0.5:1 to 3:1.

The incorporation of the compounds having formula (I) or of the mixture containing said compounds into the synthetic polymer may be accomplished according to any known procedure and in any step, before or after polymerization, or during the manufacture of the shaped article from said polymer.

Thus, for example, the powdered additives may be merely admixed, under stirring, to the polymer; or the polymer may be mixed with a solution of the stabilizers in a proper solvent, which is then evaporated; or the stabilizers can be added to the polymer at the end of the polymerization.

Furthermore, the compounds having formula (I) can be also added to the substance to be stabilized in the form of a master batch containing these compounds at a concentration ranging, for example, from 2.5 to 50% by weight.

It is furthermore possible to attain the stabilizing effect by applying the stabilizer onto the manufactured article, for example by immersing it into a stabilizer solution or dispersion and by successively evaporating the solvent or the dispersant, or by dissolving the stabilizer in a suitable solvent and spraying the solution onto the manufactured article's surface.

The following examples are given for a more detailed understanding of the present invention and for further enabling those skilled in the art to practice the same.

In the examples, unless otherwise specified, all parts are parts by weight.

Example 1

16.9 g (0.1 moles) of 2,2,6,6-tetra-methyl-4-spiro-oxirane and 21.2 g (0.1 moles) of 2,2,6,6-tetra-methyl-4-n-butyl-amino-piperidine were dissolved in 100 ml of methyl alcohol and heated, in a closed tube, at 150°C for 6 hours.

The tube was opened, the solvent was removed by evaporation, and the product obtained was distilled under vacuum at 150°C and 0.00053 da N/$cm^2$ of residual pressure.

On the basis of the I.R. and N.M.R. spectra and the elemental analyses the product was found to have formula III indicated above.

Examples 2 to 11

The reaction was conducted under the conditions of example 1. The reagents, the reaction temperature and time, the obtained compounds and the melting points thereof are listed in the following Table I.

Column 6 (obtained compound No.) refers to the list of compounds given above.

| EX. No. | REAGENTS | | Reaction time in h | Reaction temperature in °C. | Compound obtained No. | Appearance | Melting point in °C. |
|---|---|---|---|---|---|---|---|
| 2 | 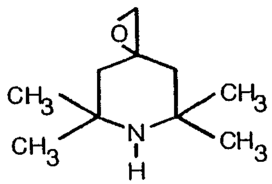 | $NH_3$ | 5 | 150 | I | white solid | 113–115 |
| 3 | ,, | 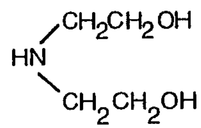 | 8 | 150 | IV | ,, | 105–107 |
| 4 | ,, | 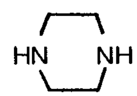 | 11 | 100 | VIII | ,, | 185–186 |
| 5 | ,, | 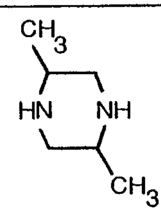 | 8 | 120 | X | ,, | 181–183 |
| 6 | ,, | 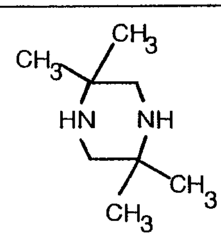 | 10 | 140 | XII | ,, | 218–220 |

| EX. No. | REAGENTS | | Reaction time in h | Reaction temperature in °C. | Compound obtained No. | Appearance | Melting point in °C. |
|---|---|---|---|---|---|---|---|
| 7 | 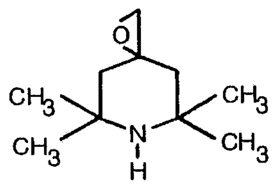 | 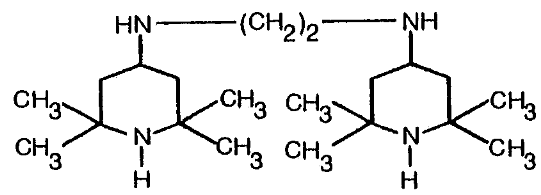 | 11 | 150 | XV | white solid | 178–180 |
| 8 | ,, | 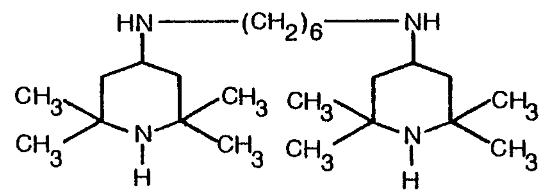 | 19 | 150 | XVI | ,, | 126–128 |
| 9 | 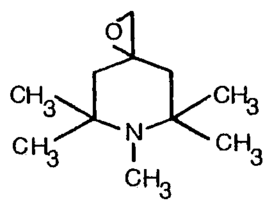 | 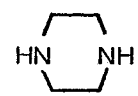 | 10 | 100 | IX | ,, | 152–153 |
| 10 | ,, | 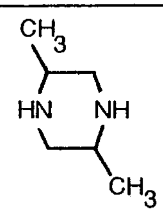 | 8 | 120 | XI | ,, | 212–214 |
| 11 | 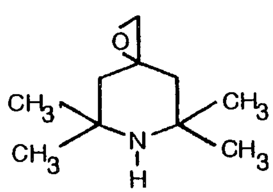 | 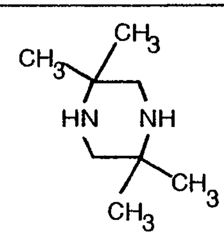 | 10 | 150 | XIII | ,, | 217–219 |

Example 12

16.9 g (0.1 mole) of 2,2,6,6-tetra-methyl-piperidyl-4-spiro-oxirane and 15 g (0.1 mole) of diethylphosphite were dissolved in 80 ml of anhydrous ethyl alcohol.

11.7 g of triethylamine were added to the mixture which was introduced into a tube. After having sealed the end of the tube, the mixture was heated at 120°C for 10 hours. The tube was opened, and the solvent, the triethyl-amine and small amounts of unreacted reagents, were removed under vacuum.

An oily, light-yellow product was obtained which, on the basis of the I.R. and N.M.R. spectra and of the elemental analyses was determined to be of formula XX shown above.

Example 13

16.9 g (0.1 mole) of 2,2,6,6-tetra-methyl-piperidyl-4-spiro-oxirane wee dissolved in 50 ml of an aqueous solution of $H_2SO_4$ (50% by weight).

The solution was allowed to stand overnight, whereupon NaOH up to a pH = 11 was added thereto, and the reaction product was extracted with $CHCl_3$. After removal of the organic solvent, a white solid product having a melting point of 130°C—132°C was obtained. On the basis of the I.R. and N.M.R. spectra and of the elemental analyses the product was determined to be of formula XXIV.

Example 14

16.9 g of 2,2,6,6-tetramethyl-piperidyl-4-spiro-oxirane and 5.5 g of sodium methylate were dissolved in 80 ml of methanol and heated in a tube sealed at its end for 10 hours at 120°C. After evaporation of the solvent, a yellow oil was obtained which, as revealed by the I.R. and N.M.R. spectra and by the elemental analyses corresponded to compound XXV indicated above.

Example 15

16.9 g of 2,2,6,6-tetramethyl-piperidyl-4-spiro-oxirane were dissolved in 40 ml of glacial acetic acid.

The solution was introduced into a tube, which was sealed at its both ends, and was heated at 120°C for 8 hours.

After neutralization with NaOH, the reaction product was distilled at 120°C and at 0.0026 da N/cm$^2$.

On the basis of the I.R. and N.M.R. spectra and of the elemental analyses, the product was determined to be of formula XXVI indicated above.

Example 16

18.7 g of the product obtained in example 13 and having formula XXIV, and 28.4 g of ethyl palmitate were molten at 160°—170°C in the presence of traces of LiOH. After removal of the ethyl alcohol formed during the reaction, the reaction product was diluted with $CH_2Cl_2$. The resulting organic solution was repeatedly washed with water up to neutrality, and the solvent was evaporated. The dried product was in the form of a light yellow oil.

On the basis of the I.R. and N.M.R. spectra end of the elemental analyses, the product was determined to be of formula XXVII above.

Example 17

The following procedure was followed in order to prove the stabilizing properties of the compounds of the present invention.

200 ml of a chloroform solution containing the stabilizing compounds to be tested, in the amount indicated in Table II, were added to 300 g of non-stabilized polypropylene having an inherent viscosity, determined in tetraline at 130°C, of 162 ml/g, a residue of the extraction with heptane of 96.5% and an ash content of 80 ppm.

The mixture was stirred for about 6 hours, at room temperature, in a rotary evaporator, then it was dried at $0.133 \cdot 10^{-4}$ da N/cm$^2$ and 50°C for 1 hour. The resulting powder was extruded in a Brabender extruder at 220°C and granulated. The granules were transformed into films having a uniform thickness of 50—60 μm and to small plates having a thickness of 1 mm.

On the manufactured articles thus obtained, the thermooxidative stability and the photo-oxidative stability were determined..

The thermo-oxidative stability was determined on the basis of the resistance to ageing in an oven, in terms of the embrittlement time (E.T.) necessary to be able to observe with the naked eye, on the examined plate, surface cracks and chalkings and other modifications due to exposure in the oven at 150°C with air circulation.

The photo-oxidative stability was determined on the basis of the embrittlement time (E.T.) in terms of the time necessary to obtain the rupture of the film by means of only one bending at 180°C, after exposure to Xenotest 1200 (manufactured by Hanau) under conditions according to standard DIN 54004:

temperature of the back panel: 43 ± 2°C
relative humidity: 50 ± 5%
alternate exposure
irradiation with light having a wave length above 300 nm.

TABLE II

| Stabilizer | | | Thermo-oxidative stability | Photo-oxidative stability |
|---|---|---|---|---|
| Example No. | Compound No. | Amount in % by weight | E.T. in hours | E.T. in hours |
| — | — | — | <24 | 100 |
| 1 | III | 0,5 | 100 | 3,400 |
| 4 | VIII | 0,5 | 200 | 2,900 |
| 6 | XII | 0,5 | 200 | 3,800 |
| 7 | XV | 0,3 | 330 | 2,800 |
| 7 | XV | 0,5 | 500 | 4,200 |
| 8 | XVI | 0,3 | 330 | 2,700 |
| 8 | XVI | 0,5 | 500 | 4,300 |
| 12 | XX | 0,5 | 170 | 3,000 |
| 16 | XVII | 0,5 | 100 | 3,100 |

**Claims**

1. Alkyl-substituted 4-methyl-piperidine derivatives of formula (I):

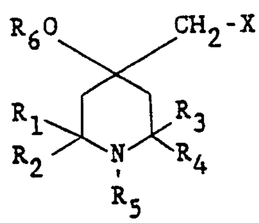

(I)

wherein:

$R_1$, $R_2$, $R_3$ and $R_4$, which may be the same or different, represent an alkyl radical having from 1 to 4 carbon atoms, or $R_1$ and $R_2$ and/or $R_3$ and $R_4$ together form an alkylene group having from 2 to 11 carbon atoms; $R_5$ is hydrogen, an alkyl radical containing from 1 to 4 carbon atoms, a hydroxyalkyl or alkoxy radical containing from 1 to 6 carbon atoms, an aryl or aryl-alkyl radical containing from 6 to 18 carbon atoms, an alkenyl radical having from 2 to 6 carbon atoms, or the group:

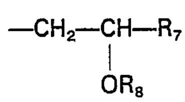

a)

wherein

$R_7$ is hydrogen, an alkyl radical containing from 1 to 4 carbon atoms or an alkoxy radical containing from 1 to 6 carbon atoms, and

$R_8$ is hydrogen, an alkyl radical containing from 1 to 4 carbon atoms, or the group:

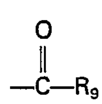

b)

wherein

$R_9$ is hydrogen, an alkyl radical containing from 1 to 18 carbon atoms, a phenyl or benzyl radical or the group:

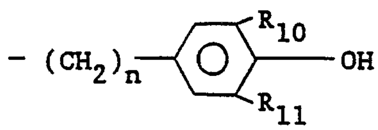 c)

wherein *n* is an integer from 1 to 10, and $R_{10}$ and $R_{11}$ is hydrogen or an alkyl radical containing from 1 to 4 carbon atoms;

$R_6$ is hydrogen, an alkyl radical, an alkyl-carbonyl radical, in which the alkyl group contains from 1 to 22 carbon atoms, a phenyl-alkyl-carbonyl radical, a cyclo-alkyl-carbonyl radical or a phenyl-carbonyl radical, in which the phenyl or cycloalkyl nucleus is optionally substituted by alkyl groups containing from 1 to 3 carbon atoms; and

X may be:

A) $-NR_{12}R_{13}$, in which $R_{12}$ and $R_{13}$, which may be the same or different from each other, represent:

an alkyl radical containing from 1 to 10 carbon atoms;

an alkenyl or alkynyl radical containing from 2 to 10 carbon atoms;

benzyl, phenyl or the corresponding alkyl-substituted radicals;

the radical:

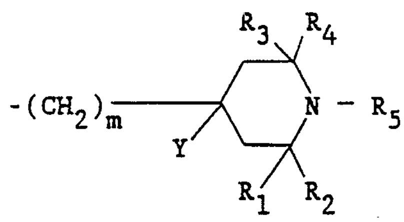 d)

wherein:

$R_1$, $R_2$, $R_3$, $R_4$ and $R_5$ have the meaning indicated hereinbefore, *m* is 0 or an integer from 1 to 3 and Y is hydrogen, a hydroxyl group, a hydroxyalkyl-carbonyl radical, a hydroxy-phenyl-carbonyl radical, a hydroxy-cycloalkyl-carbonyl radical, in which the phenyl or cycloalkyl group may be alkyl-substituted;

the radical:

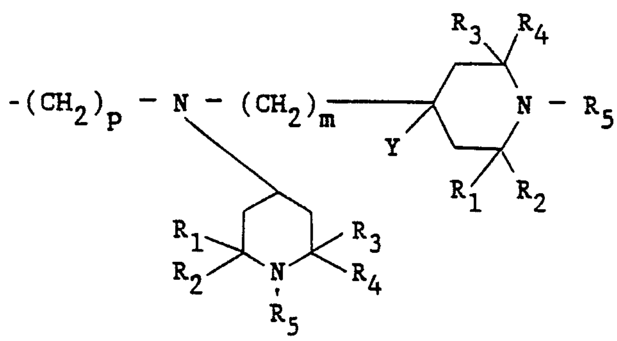 e)

wherein:

$R_1$, $R_2$, $R_3$, $R_4$, $R_5$, m and Y have the same meaning explained hereinbefore and p is an integer ranging from 1 to 12;

the radical

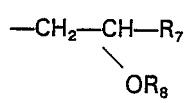 f)

wherein:

$R_7$ and $R_8$ have the meanings indicated hereinabove;

the triazine radical

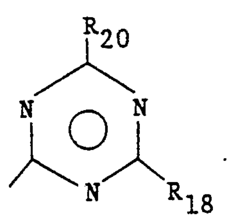 g)

wherein

$R_{20}$ and $R_{18}$, which may be the same or different, each represent an alkyl radical containing from 1 to 18

carbon atoms, an alkoxy radical containing from 1 to 8 carbon atoms, or the radical:

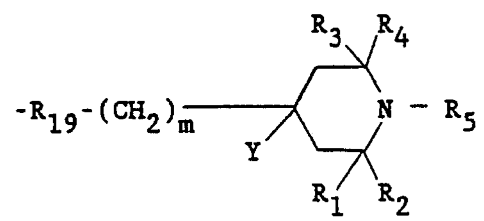

wherein:

$R_1$, $R_2$, $R_3$, $R_4$, $R_5$, m and Y have the meanings as indicated hereinbefore and $R_{19}$ may be oxygen or —N—$R_{12}$; or

$R_{12}$ and $R_{13}$ form, along with the nitrogen atom they are linked to, an unsubstituted or akyl-substituted morpholino or piperidino group; or

$R_{12}$ and $R_{13}$ together form the group:

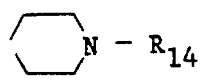 h)

unsubstituted or alkyl-substituted in the nucleus, in which $R_{14}$ is hydrogen, an alkyl radical containing from 1 to 6 carbon atoms, or the radical:

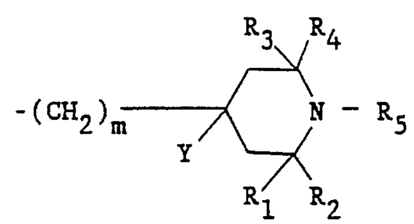 i)

wherein

m, $R_1$, $R_2$, $R_3$, $R_4$, $R_5$ and Y have the meanings indicated hereinbefore; or

$R_{12}$ or $R_{13}$ may be hydrogen, with the proviso that neither is hydrogen when radical g) is present;

B)

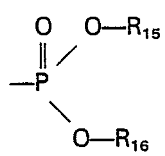

in which $R_{15}$ and $R_{16}$, which may be the same or different from each other, each represent hydrogen, an alkyl radical containing from 1 to 6 carbon atoms, or the group:

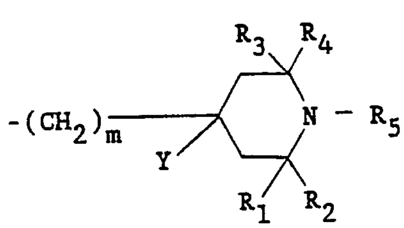

in which

$R_1$, $R_2$, $R_3$, $R_4$, $R_5$, Y and m have the meaning indicated hereinbefore; or

C) —O—$R_{17}$, in which $R_{17}$ may be:

hydrogen;

an alkyl, alkenyl or alkynyl radical, each containing from 1 to 18 carbon atoms;

a benzyl or phenyl radical, optionally substituted in the nucleus;

the radical:

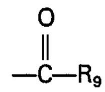 b)

in which $R_9$ has the meaning indicated hereinabove; or

the triazine radical

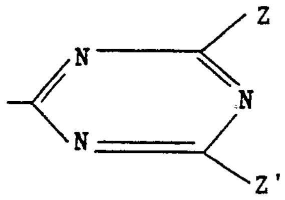 n)

in which:

Z and Z', which may be the same or different, each represent hydrogen, an alkyl radical containing from 1 to 18 carbon atoms, an alkoxy or alkylamino group in which the alkyl radical contains from 1 to 18 carbon atoms, or the radical:

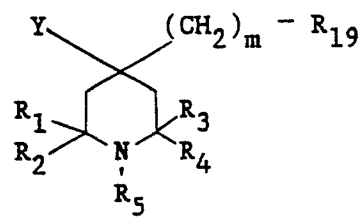

in which

$R_1$, $R_2$, $R_3$, $R_4$, $R_5$, $R_{19}$, Y and m have the meanings indicated hereinbefore.

2. Alkyl-substituted 4-methyl-piperidine derivatives according to claim 1, in which $R_1$, $R_2$, $R_3$, $R_4$ are methyl or ethyl radicals.

3. Alkyl-substituted 4-methyl-piperidine derivatives according to claims 1 or 2, wherein $R_5$ is hydrogen, a methyl radical or an allyl radical.

4. Alkyl-substituted 4-methyl-piperidine derivatives according to any one of the preceding claims, wherein $R_6$ is hydrogen, a methyl or an ethyl radical.

5. Alkyl-substituted 4-methyl-piperidine derivatives according to any one of the preceding claims wherein X is selected from

A) $-NR_{12}R_{13}$ in which

$R_{12}$ and $R_{13}$ both represent an alkyl radical containing from 1 to 10 carbon atoms; or

one of $R_{12}$ and $R_{13}$ represents the radical d) as defined in claim 1 ($m = 0$) and the other one is an alkyl radical containing from 1 to 10 carbon atoms; or

at least one of $R_{12}$ and $R_{13}$ is the radical e) as defined in claims 1; or

$R_{12}$ and $R_{13}$ together form the alkyl-substituted group h) as defined in claim 1; or

at least one of $R_{12}$ and $R_{13}$ represents the radical g) as defined in claim 2 wherein $R_{18}$ and $R_{20}$ both are alkoxy radicals containing from 1 to 18 carbon atoms;

B)

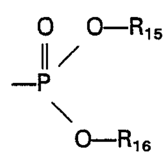

in which

$R_{15}$ and $R_{16}$ are as defined in claim 1;

C) $-O-R_{17}$ in which $R_{17}$ may be

an alkyl radical having from 1 to 18 carbon atoms; or

the radical n) as defined in claim 1 in wherein Z and Z' both are either alkoxy groups in which the akyl radical contains from 1 to 18 carbon atoms or represent the radical

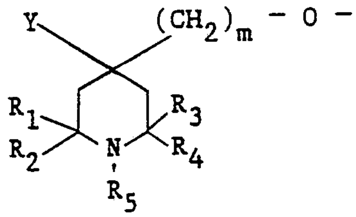

wherein

$R_1$, $R_2$, $R_3$, $R_4$, $R_5$, Y and m are as defined in the preceding claims.

6. A process for synthesizing the derivatives of alkyl-substituted 4-methyl-piperidines of formula (I), according to claim 1, wherein X is the $—NR_{12}R_{13}$ group, consisting in reacting, in the presence of an inert solvent and at a temperature ranging from room temperature to 200°C, an alkyl-piperidyl-4-spiro-oxirane with an equimolar amount of a primary or secondary amine.

7. A process for synthesizing the derivatives of alkyl-substituted 4-methyl-piperidines of formula (I), according to claim 1, wherein X is the

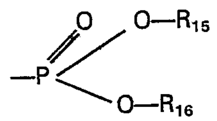

group, consisting in reacting, in the presence of an inert solvent and at a temperature ranging from room temperature to 200°C, an alkyl-piperidyl-4-spiro-oxirane with an equimolar amount of a phosphite.

8. A process for synthesizing the derivatives of alkyl-substituted 4-methyl-piperidines of formula (I), according to claim 1, wherein X is the $—O—R_{17}$ group, consisting in reacting, in the presence of an organic solvent and at a temperature ranging from room temperature to 200°C, an alkyl-piperidyl-4-spiro-oxirane with an equimolar amount of an alcoholate of an alkali or alkaline-earth metal.

9. A process for synthesizing the derivatives of alkyl-substituted 4-methyl-piperidines of formula (I), according to claim 1, wherein X is $—O—R_{17}$ and $R_{17}$ is

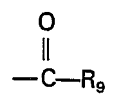

b)

or the triazine radical (n), consisting in reacting the compound of formula XXIV

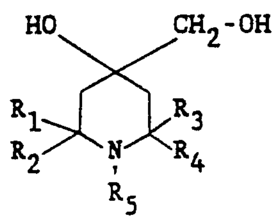

(XXIV)

with an halogen derivative containing an acyl or triazinyl-radical, in the presence of a base and optionally of an inert solvent, at a temperature ranging from room temperature to 200°C.

10. A composition comprising a synthetic organic polymeric substance stabilized against light, oxygen and heat characterized in that it contains, in an amount sufficient to prevent any degradation of the polymer, a derivative of an alkyl-substituted 4-methyl-piperidine having formula (I) of claim 1.

11. A composition according to claim 10, characterized in that the derivative of the alkyl-substituted 4-methyl-piperidine of general formula (I) is present in an amount ranging from 0.01 to 5% by weight based on the synthetic organic polymer substance.

12. A composition according to claim 10, characterized in that the synthetic polymeric substance is a polyolefin and preferably polypropylene prevailingly consisting of isotactic macromolecules.

13. A composition according to claim 12, characterized in that the derivative of the alkyl-substituted 4-methyl-piperidine of general formula (I) is contained therein in an amount ranging from 0.01 to 2% by weight.

14. A composition according to claim 10, characterized in that the synthetic polymeric substance is a halogen-containing vinyl polymer.

15. A composition according to claim 14, characterized in that the alkyl-substituted 4-methyl-piperidine derivative of general formula (I) is contained therein in an amount ranging from 0.01 to 1% by weight with respect to the halogen-containing vinyl-polymer.

16. A composition according to claim 10, characterized in that the synthetic polymeric substance is a polyurethane, a polyamide or a polymer derived from an α, β unsaturated acid.

17. A composition according to claim 16, characterized in that the alkyl-substituted 4-methyl-piperidine derivative of general formula (I) is contained therein in an amount ranging from 0.01 go 5% by weight with respect to the polymeric substance.

18. A composition according to any one of claims 10 to 17, characterized in that the alkyl-substituted 4-methyl-piperidine derivative of general formula (I) is employed in admixture with other additives selected from antioxidants, UV-ray absorbers, pigments, fillers, basic nitrogen containing polycondensates and stabilizers.

19. A composition according to claim 18, characterized in that the other additives are employed, along with the alkyl-substituted 4-methyl-piperidine derivative of general formula (I), in a weight ratio ranging from 0.5:1 to 3:1.

**Patentansprüche**

1. Alkylsubstituierte 4-Methyl-piperidin-derivate der Formel I

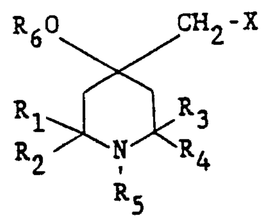

(I)

worin

$R_1$, $R_2$, $R_3$ und $R_4$, die gleich oder verschieden sein können, einen Alkylrest mit 1 bis 4 C-Atomen bedeuten, oder $R_1$ und $R_2$ und/oder $R_3$ und $R_4$ bilden gemeinsam eine Alkylgruppe mit 2 bis 11 C-Atomen; $R_5$ ist Wasserstoff, ein Alkylrest mit 1 bis 4 C-Atomen, ein Hydroxyalkyl- oder Alkoxyrest mit 1 bis 6 C-Atomen, ein Aryl- oder Arylalkylrest mit 6 bis 18 C-Atomen, ein Alkenylrest mit 1 bis 6 C-Atomen oder die Gruppe

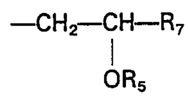

a)

worin

$R_7$ Wasserstoff, ein Alkylrest mit 1 bis 4 C-Atomen oder ein Alkoxyrest mit 1 bis 6 C-Atomen ist und $R_8$ Wasserstoff, ein Alkylrest mit 1 bis 4 C-Atomen oder die Gruppe

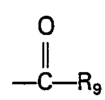

b)

worin

$R_9$ Wasserstoff, ein Alkylrest mit 1 bis 18 C-Atomen, ein Phenyl- oder Benzylrest oder die Gruppe

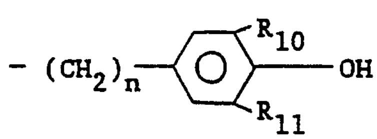

c)

ist, worin n eine ganze Zahl von 1 bis 10 ist und $R_{10}$ und $R_{11}$ Wasserstoff oder ein Alkylrest mit 1 bis 4 C-Atomen sind;

$R_6$ ist Wasserstoff, ein Alkylrest, ein Alkylcarbonylrest, in welchem die Alkylgruppe 1 bis 22 C-Atome enthält, ein Phenylalkylcarbonylrest, ein Cycloalkylcarbonylrest oder ein Phenylcarbonylrest, in welchem der Phenyl- oder Cycloalkylkern gegebenenfalls durch Alkylgruppen mit 1 bis 3 C-Atomen substituiert ist, und X bedeuten kann:

A) —$NR_{12}R_{13}$, worin $R_{12}$ und $R_{13}$, die gleich oder verschieden ein können, bedeuten:

einen Alkylrest mit 1 bis 10 C-Atomen;

einen Alkenyl- oder Alkinylrest mit 2 bis 10 C-Atomen;

Benzyl-, Phenyl- oder die entsprechenden alkylsubstituierten Reste;

den Reste:

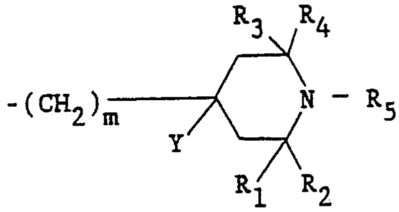

d)

worin

$R_1$, $R_2$, $R_3$, $R_4$ und $R_5$ die oben angegebenen Bedeutung haben, m = 0 oder eine ganze Zahl von 1 bis 3 ist, und Y Wasserstoff, eine Hydroxylgruppe, eine Hydroxyalkylcarbonylrest, ein Hydroxyphenylcarbonylrest, ein Hydroxycycloalkylcarbonylrest, in welchem die Phenyl- oder Cycloalkylgruppe alkylsubstituiert sein kann, ist;

den Rest

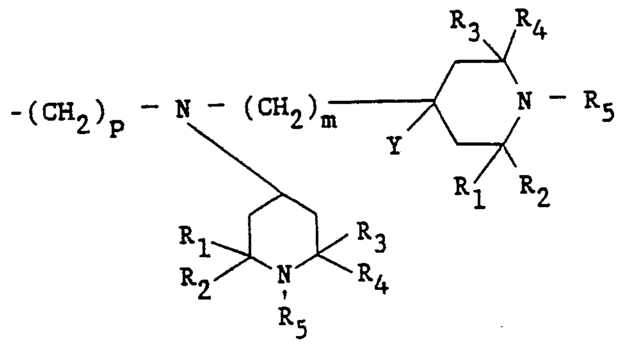

e)

worin

$R_1$, $R_2$, $R_3$, $R_4$, $R_5$, m und Y die oben erläuterte Bedeutung haben und p eine ganze Zahl von 1 bis 12 ist;

den Rest

$$—CH_2—CH—R_7$$
$$|$$
$$OR_8$$

f)

worin

$R_7$ und $R_8$ die obige Bedeutung haben;

den Triazinrest

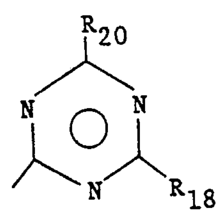

g)

worin

$R_{20}$ und $R_{18}$, die gleich oder verschieden sein können, jeweils einen Alkylrest mit 1 bis 18 C-Atomen, einen Alkoxyrest mit 1 bis 18 C-Atomen oder den Rest

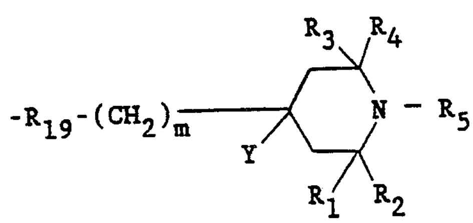

bedeuten, worin $R_1$, $R_2$, $R_3$, $R_4$, $R_5$, m und Y die oben angegebene Bedeutung haben und $R_{19}$ Sauerstoff oder —N—$R_{12}$ sein kann; oder

$R_{12}$ und $R_{13}$ bilden zusammen mit dem Stickstoffatom, an das sie gebunden sind, eine unsubstituierte oder alkylsubstituierte Morpholin oder Piperidingruppe; oder

$R_{12}$ und $R_{13}$ bilden gemeinsam die Gruppe

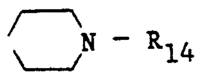

h)

die im Kern unsubstituiert oder alkylsubstituiert sein kann und in welcher $R_{14}$ Wasserstoff, ein Alkylrest mit 1 bis 6 C-Atomen oder der Rest

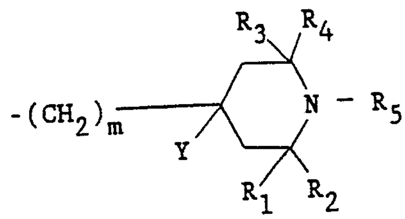

i)

sein kann, worin m, $R_1$, $R_2$, $R_3$, $R_4$, $R_5$ und Y die oben angegebene Bedeutung haben; oder

$R_{12}$ oder $R_{13}$ können Wasserstoff sein, unter der Bedingung, daß keiner der Reste Wasserstoff ist, wenn der Rest g) vorliegt;

B)

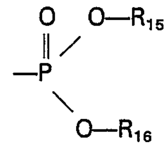

worin

$R_{15}$ und $R_{16}$, die gleich oder verschieden sein können, jeweils Wasserstoff, einen Alkylrest mit 1 bis 6 C-Atomen oder die Gruppe

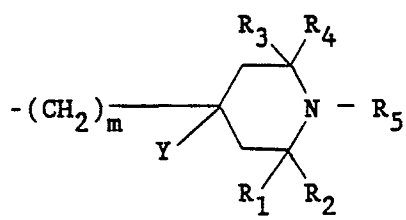

bedeuten, in welcher $R_1$, $R_2$, $R_3$, $R_4$, $R_5$, Y und m die oben angegebene Bedeutung haben; oder

C) —O—$R_{17}$, worin $R_{17}$ sein kann:

Wasserstoff;

ein Alkyl-, Alkenyl- oder Alkinylrest, jeweils mit 1 bis 18 C Atomen;

ein Benzyl- oder Phenylrest, gegebenenfalls am Kern substituiert;

der Rest

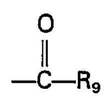

b)

worin $R_9$ die oben angegebe Bedeutung hat; oder

der Triazinrest

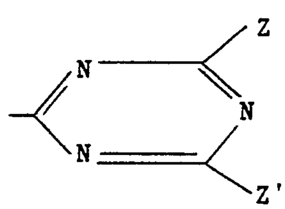

n)

worin Z und Z', die gleich oder verschieden sein können, jeweils Wasserstoff, einen Alkylrest mit 1 bis 18 C-Atomen, eine Alkoxy- oder Alkylaminogruppe, in welcher der Alkylrest 1 bis 18 C-Atome enthält, oder den Rest

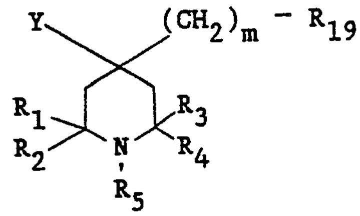

bedeuten, in welchem $R_1$, $R_2$, $R_3$, $R_4$, $R_5$, $R_{19}$, Y und m die oben angegebene Bedeuten haben.

2. Alkylsubstituierte 4-Methyl-piperidin-derivate nach Anspruch 1, in welchen $R_1$, $R_2$, $R_3$, $R_4$ Methyl- oder Ethylreste sind.

3. Alkylsubstituierte 4-Methyl-piperidin-derivate nach Anspruch 1 oder 2, worin $R_5$ Wasserstoff, ein Methylrest oder ein Allylrest ist.

4. Alkylsubstituierte 4-Methyl-piperidin-derivate nach irgendeinem der vorhergehenden Ansprüche, worin $R_6$ Wasserstoff, ein Methyl- oder Ethylrest ist.

5. Alkylsubstituierte 4-Methyl-piperidin-derivate nach irgendeinem der vorhergehenden Anspruche, worin X ausgewählt is aus:

A) $—NR_{12}R_{13}$, worin

$R_{12}$ und $R_{13}$ beide einen Alkylrest mit 1 bis 10 C-Atomen bedeuten; oder

eine der Substituenten $R_{12}$ und $R_{13}$ steht für den Rest d) gemäß Definition in Anspruch 1 (m = 0) und der andere ist ein Alkylrest mit 1 bis 10 C-Atomen; oder

mindestens einer der Substituenten $R_{12}$ und $R_{13}$ ist der Rest e) demäß Definition in Anspruch 1; oder $R_{12}$ und $R_{13}$ bilden gemeinsam die alkylsubstituierte Gruppe h) gemäß Definition in Anspruche 1; oder

mindestens einer der Substituenten $R_{12}$ und $R_{13}$ bedeutet den Rest g) gemäß Definition in Anspruch 1, worin $R_{18}$ und $R_{20}$ jeweils Alkoxyreste mit 1 bis 18 C-Atomen sind;

B)

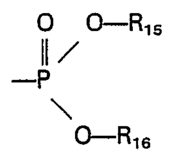

worin

$R_{15}$ und $R_{16}$ wie in Anspruch 1 definiert sind;

C) $—O—R_{17}$, worin $R_{17}$ sein kann;

ein Alkylrest mit 1 bis 8 C-Atomen; oder

der Rest n gemäß Definition in Anspruch 1, worin Z und Z' beide jeweils eine Alkoxygruppe, in welcher der Alkylrest 1 bis 18 C-Atome enthält, oder der Rest

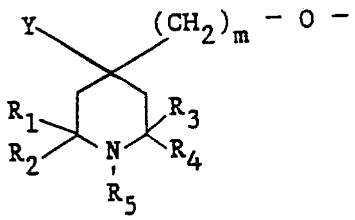

sind, worin $R_1$, $R_2$, $R_3$, $R_4$, $R_5$, Y und m wie in den vorhergehenden Ansprüchen definiert sind.

6. Verfahren zur Herstellung der Derivate der alkylsubstituierten 4-Methyl-piperidine der Formel (I) gemäß Anspruch 1, worin X die Gruppe $—NR_{12}R_{13}$ ist, bestehend aus der Umsetzung eines Alkyl-piperidyl-4-spiro-oxirans mit einer äquimolaren Menge eines primären oder sekundären Amins in Anwesenheit eines inerten Lösungsmittels und bei einer Temperatur zwischen Raumtemperatur und 200°C.

7. Verfahren zur Herstellung der Derivate der alkylsubstituierten 4-Methyl-piperidine der Formel (I) gemäß Anspruch 1, worin X die Gruppe

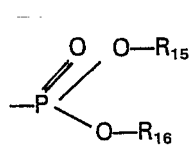

ist, bestehend aus der Umsetzung eines Alkyl-piperidyl-4-spiro-oxirans mit einer äquimolaren Menge eines Phosphits in Anwesenheit eines inerten Lösungsmittels und bei einer Temperatur zwischen Raumtemperatur bis 200°C.

8. Verfahren zur Herstellung der Derivate der alkylsubstituierten 4-Methyl-piperidine der Formel (I)

gemäß Anspruch 1, worin X die Gruppe —O—$R_{17}$ ist, bestehende aus der Umsetzung eines Alkyl-piperidyl-4-spiro-oxirans mit einer äquimolaren Menge eines Alkoholates eines Alkali- oder Erdalkalimetalls in Anwesenheit eines organischen Lösungsmittels und bei einer Temperatur zwischen Raumtemperatur bis 200°C.

9. Verfahren zur Herstellung der Derivate der alkylsubstituierten 4-Methyl-piperidine der Formel (I) gemäß Anspruch 1, worin X —O—$R_{17}$ ist und $R_{17}$

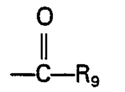

b)

oder den Triazinrest (n) bedeutet, bestehend aus der Umsetzung der Verbindung der Formel XXIV

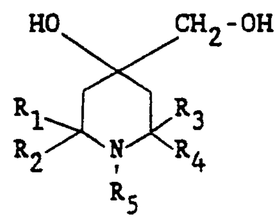

mit einem Halogenderivat, enthaltend einen Acyl- oder Triazinylrest, in Anwesenheit einer Base und gegebenenfalls eines inerten Lösungsmittels bei Temperatur zwischen Raumtemperatur bis 200°C.

10. Zusammensetzung, enthaltend eine synthetische, organische, polymere Substanz, die gegen Licht, Sauerstoff und Wärme stabilisiert ist, dadurch gekennzeichnet, daß sie in einer zur Verhütung von jeglicher Zersetzung des Polymers aureichenden Menge ein Derivat eines alkylsubstituierten 4-Methyl-piperidins der Formel (I) von Anspruch 1 enthält.

11. Zusammensetzung nach Anspruch 10, dadurch gekennzeichnet, daß das Derivat des alkylsubstituierten 4-Methyl-piperidins der allgemeinen Formel (I) in einer Menge von 0,01 bis 5 Gew.-%, bezogen auf die synthetische, organische, polymere Substanz, anwesend ist.

12. Zusammensetzung nach Anspruch 10, dadurch gekennzeichnet, daß die synthetische, polymere Substanz ein Polyolefin und vorzugsweise Polypropylen, das hauptsächlich aus isotaktischen Makromolekülen besteht, ist.

13. Zusammensetzung nach Anspruch 12, dadurch gekennzeichnet, daß sie das Derivat des alkylsubstituierten 4-Methyl-piperidins der allgemeinen Formel (I) in einer Menge von 0,01 bis 2 Gew.-% enthält.

14. Zusammensetzung nach Anspruch 10, dadurch gekennzeichnet, daß die synthetische, polymere Substanz ein halogenhaltiges Vinylpolymer ist.

15. Zusammensetzung nach Anspruch 14, dadurch gekennzeichnet, daß sie das alkylsubstituierte 4-Methyl-piperidin-derivat der allgemeinen Formel (I) in einer Menge von 0,01 bis 1 Gew.-%, bezogen auf das halogenhaltige Vinylpolymer, enthält.

16. Zusammensetzung nach Anspruch 10, dadurch gekennzeichnet, daß die synthetische polymere Substanz ein Polyurethan, ein Polyamid oder ein von einer α,β-ungesättigten Säure abgeleitetes Polymer ist.

17. Zusammensetzung nach Anspruch 16, dadurch gekennzeichnet, daß sie das alkylsubstituierte 4-Methyl-piperidin-derivat der allgemeinen Formel (I) in eine Menge von 0,01 bis 5 Gew.-%, bezogen auf die polymere Substanz, enthält.

18. Zusammensetzung nach irgendeinem der Anspruch 10 bis 17, dadurch gekennzeichnet, daß das alkylsubstituierte 4-Methyl-piperidin-derivat der allgemeinen Formel (I) in Mischung mit anderen Zusätzen, ausgewählt aus Antioxidationsmitteln, UV-Absorptionsmitteln, Pigmenten, Füllmitteln, basischen Stickstoff enthaltenden Polykondensaten und Stabilisatoren, verwendet wird.

19. Zusammensetzung nach Anspruch 18, dadurch gekennzeichnet, daß die anderen Zusätze zusammen mit dem alkylsubstituierten 4-Methyl-piperidin-derivat der allgemeinen Formel (I) in einem Gewichtsverhältnis von 0,5:1 bis 3:1 verwendet werden.

**Revendications**

1. Dérivés d'alkyl-4-méthyl-pipéridine de formule (I):

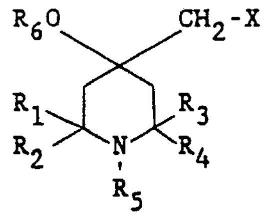

(I)

dans laquelle:

$R_1$, $R_2$, $R_3$ et $R_4$ qui peuvent être identiques ou différents, représentent un radical alkyle renfermant de 1 à 4 atomes de carbone, ou $R_1$ et $R_2$ et/ou $R_3$ et $R_4$ pris ensemble forment une groupe alkylène renfermant de 2 à 11 atomes de carbone;

$R_5$ représente un atome d'hydrogène, un radical alkyle contenant de 1 à 4 atomes de carbone, un radical hydroxyalkyle ou alkoxy contenant de 1 à 4 atomes de carbone, un radical aryle ou arylalkyle contenant de 6 à 18 atomes de carbone, un radical alkényle renfermant de 2 à 6 atomes de carbone, ou le groupe:

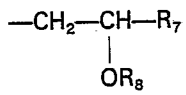

a)

dans lequel;

$R_7$ représente un atome d'hydrogène, un radical alkyle contenant de 1 à 4 atomes de carbone ou un radical alkoxy contenant de 1 à 6 atomes de carbone; et

$R_8$ représente un atome d'hydrogène, un radical alkyle contenant de 1 à 4 atomes de carbone, ou le groupe

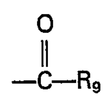

b)

dans lequel:

$R_9$ représent un atome d'hydrogène, un radical alkyle contenant de 1 à 18 atomes de carbone, un radical phényle ou benzyle,

ou le groupe:

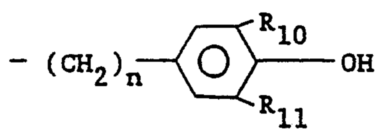

c)

dans lequel:

n représente un nombre entier comprise entre 1 et 10; et

$R_{10}$ et $R_{11}$ représentent un atome d'hydrogène ou un radical alkyle contenant de 1 à 4 atomes de carbone;

$R_6$ représente un atome d'hydrogène, un radical alkyle, un radical alkylcarbonyle dans lequel le groupe alkyle contient de 1 à 22 atomes de carbone, un radical phényl-alkyl-carbonyle, un radical cycloalkylcarbonyle ou un radical phénylcarbonyle dans lequel le noyau phényle ou cycloalkyle est éventuellement substitué par des groupes alkyles contenant de 1 à 3 atomes de carbone; et

X peut représenter:

A) $-NR_{12}R_{13}$ dans lequel $R_{12}$ et $R_{13}$, qui peuvent être identiques ou différents l'un de l'autre, représentent:

un radical alkyle contenant de 1 à 10 atomes de carbone,

un radical alkényle ou alkynyle contenant de 2 à 10 atomes de carbone;

les radicaux benzyle, phényle ou les dérivés alkyles substitués correspondants;

le radical:

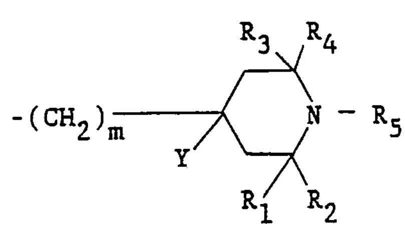

d)

dans lequel:

$R_1$, $R_2$, $R_3$, $R_4$ et $R_5$ ont les mêmes significations qui'indiquées ci-dessus;

m est égal à 0 ou représente un nombre entier de 1 à 3; et

Y représente un atome d'hydrogène, un group hydroxyle, un radical hydroxyalkylcarbonyle, un radical hydroxyphénylcarbonyle, un radical hydroxycycloalkylcarbonyle dans lequel le groupe phényle ou cycloalkyle peut être alkyle substitué;

le radical:

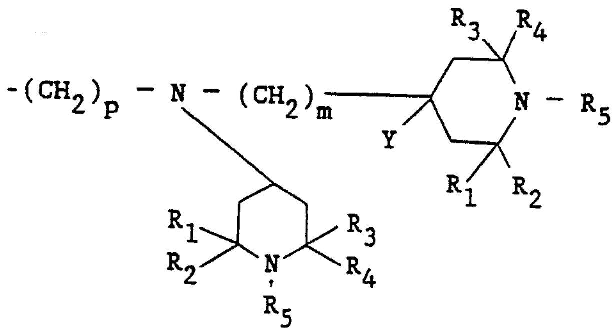

e)

dans lequel:

$R_1$, $R_2$, $R_3$, $R_4$, $R_5$, m et Y ont les mêmes significations qu'indiquées ci-dessus; et

p représente un nombre entier compris entre 1 et 12 le radical:

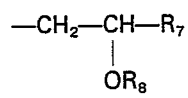

f)

dans lequel $R_7$ et $R_8$ ont les significations indiquées ci-dessus;

le radical triazine:

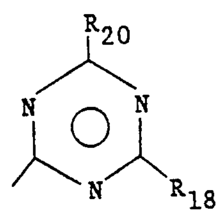

g)

dans lequel:

$R_{20}$ et $R_{18}$, qui peuvent être identiques ou différents, repésentent chacun un radical alkyle contenant de 1 à 18 atomes de carbone; un radical alkoxy contenant de 1 à 18 atomes de carbone,

ou radical:

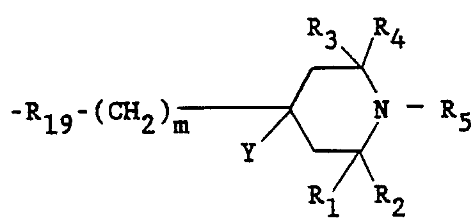

dans lequel;

$R_1$, $R_2$, $R_3$, $R_4$, $R_5$, m et Y ont les significations indiquées ci-dessus; et

$R_{19}$ peut représenter un atome d'oxygène ou —N—$R_{12}$, ou

$R_{12}$ et $R_{13}$ forment en même temps que l'atome d'hydrogène auquel ils sont liés, un groupe morpholino ou pipéridino non substitué ou substitué par un radical alkyle; ou

$R_{12}$ et $R_{13}$ forment ensemble le group:

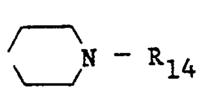

h)

non substitué ou substitué par un radical alkyle dans le noyau, dans lequel $R_{14}$ représente un atome d'hydrogène, un radical alkyle contenant de 1 à 6 atomes de carbone, ou le radical

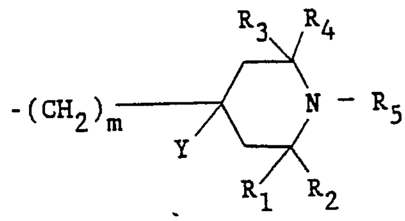 i)

dans lequel m, $R_1$, $R_2$, $R_3$, $R_5$ et Y ont les significations indiquées ci-dessus; ou

$R_{12}$ ou $R_{13}$ peuvent représenter un atome d'hydrogène, sous réserve que ni l'un ni l'autre ne représente un atome d'hydrogène lorsque le radical g) est présent;

B)

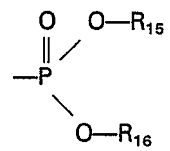

dans lequel:

$R_{15}$ et $R_{16}$, qui peuvent être identiques ou différents l'un de l'autre, représentent chacun un atome d'hydrogène, un radical alkyle contenant de 1 à 6 atomes de carbone ou le groupe:

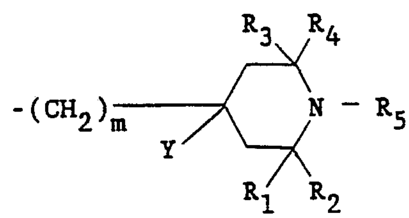

dans lequel $R_1$, $R_2$, $R_3$, $R_4$, $R_5$, Y et m ont les significations indiquées ci-dessus; ou

C) —O—$R_{17}$ dans lequel $R_{17}$ peut représenter:

un atome d'hydrogène;

un radical alkyle, alkényle ou alkynyl renfermant chacun de 1 à 8 atomes de carbone;

le radical benzyl ou phényle, éventuellement substitué dans le noyau;

le radical:

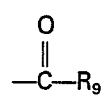 b)

dans lequel $R_9$ présente la signification indiquée ci-dessus; ou

le radical triazine

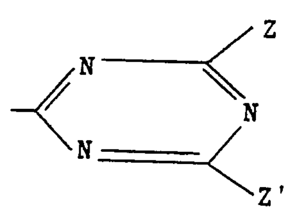 n)

dans lequel: Z et Z', qui peuvent être identiques ou différentes, représentent chacun un atome d'hydrogène, un radical alkyle contenant de 1 à 18 atomes de carbone, un groupe alkoxy ou alkylamino dans lequel le radical alkyle contient de 1 à 18 atomes de carbone,

ou le radical:

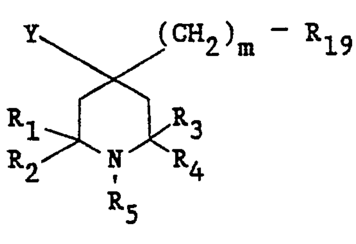

dans lequel $R_1$, $R_2$, $R_3$, $R_4$, $R_5$, $R_{19}$, Y et m ont les significations indiquées ci-dessus.

2. Dérivés d'alkyl-4-méthyl-pipéridine selon la revendication 1, dans lesquels $R_1$, $R_2$, $R_3$, $R_4$ représentent des radicaux méthyle ou éthyle.

3. Dérivés d'alkyl-4-méthyl-pipéridine selon la revendication 1 ou 2, dans lesquels $R_5$ représente un atome d'hydrogène, le radical méthyle ou le radical allyle.

4. Dérivés d'alkyl-4-méthyl-pipéridine selon une quelconque des revendications précédentes, dans lesquels $R_6$ représente un atome d'hydrogène, le radical méthyle ou le radical éthyle.

5. Dérivés d'alkyl-4-méthyl-pipéridine selon une quelconque des revendications précédentes, dans lesquels X est choisi parmi:

A) $—NR_{12}R_{13}$ dans lequel:

$R_{12}$ et $R_{13}$ représentent tous les deux un radical alkyle contenant de 1 à 10 atomes de carbone; ou

l'un d'entre $R_{12}$ et $R_{13}$ représente le radical d) tel que défini dans la revendication 1 (m = 0) et l'autre représente un radical alkyle renfermant de 1 à 10 atomes de carbone; ou

au moins l'un d'entre $R_{12}$ et $R_{13}$ représente le radical e) tel que défini dans la revendication 1; ou

$R_{12}$ et $R_{13}$ forment ensemble le groupe h) substitué par un radical alkyle tel que défini dans la revendication 1; ou

au moins l'un d'entre $R_{12}$ et $R_{13}$ représente le radical g) tel que défini dans la revendication 1, dans lequel $R_{18}$ et $R_{20}$ représentent tous deux des radicaux alkoxy contenant de 1 à 18 atomes de carbone;

B)

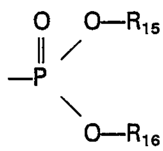

dans lequel $R_{15}$ et $R_{16}$ sont tels que définis dans la revendication 1;

C) $—O—R_{17}$ dans lequel $R_{17}$ peut être:

un radical alkyle renfermant de 1 à 18 atomes de carbone; ou

le radical n) tel que défini dans la revendication 1 dans lequel Z et Z' représentent tous deux soit des groupes alkoxy dans lesquels le radical alkyle contient de 1 à 18 atomes de carbone, soit le radical:

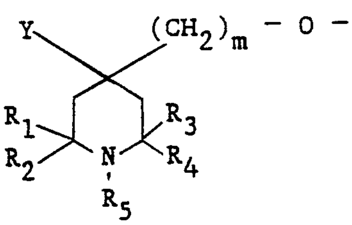

dans lequel $R_1$, $R_2$, $R_3$, $R_4$, $R_5$, Y et m sont tels que définis dans les revendications précédentes.

6. Un procédé de synthèse des dérivés d'alkyl-4 méthyl-pipéridine de formule (I) selon la revendication 1, dans lequel X représente le groupe $—NR_{12}R_{13}$, consistant à faire réagir, en présence d'un solvant inerte et à une température comprise entre la température ambiante et 200°C, un alkyl-pipéridyl-4-spiro-oxirane avec une quantité équimolaire d'une amine primaire ou secondaire.

7. Un procédé de synthèse de dérivés d'alkyl-4-méthyl-pipéridine de formule (I) selon la revendication 1, dans lesquels X représente le groupe:

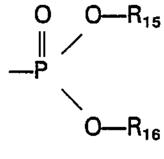

consistant à faire réagir, en présence d'un solvant inerte et à une température comprise entre la température ambiante et 200°C, un alkyl-pipéridyl-4-spiro-oxirane avec une quantité équimolaire d'un phosphite.

8. Un procédé de synthèse des dérivés d'alkyl-4-méthyl-pipéridine de formule (I) selon la revendication 1, dans lesquels X représente le groupe $—O—R_{17}$, consistant à faire réagir, en présence d'un solvant organique et à une température comprise entre la température ambiante et 200°C, un alkyl-pipéridyl-4-spiro-oxirane avec une quantité équimolaire d'un alcoolate d'un métal alcalin ou alcalino-terreux.

9. Un procédé de synthèse des dérivés d'alkyl-4-méthyl-pipéridine de formule (I) selon la revendication 1, dans lesquels X représente $—O—R_{17}$ et $R_{17}$ représente:

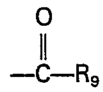 (b)

ou le radical triazine n), consistant à faire réagir le dérivé de formule XXIV:

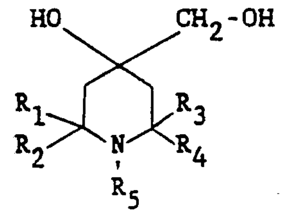

avec un dérivé halogéné contenant un radical acyle ou triazinyle, en présence d'une base et éventuellement d'un solvant inerte, à une température comprise entre la température ambiante et 200°C.

10. Une composition comprenant une substance polymère organique synthétique stabilisée à la lumière, à l'oxygène et à la chaleur, caractérisée en ce qu'elle contient, en quantité suffisante pour empêcher toute dégradation du polymère, un dérivé d'une alkyl-4-méthyl-pipéridine de formule (I) selon la revendication 1.

11. Une composition selon la revendication 10, caractérisée en ce que le dérivé de l'alkyl-4-méthyl-pipéridine de formule générale (I) est présent en quantité comprise entre 0,01 et 5% en poids par rapport au poids de la substance polymère organique synthétique.

12. Une composition selon la revendication 10, caractérisée en ce que la substance polymère synthétique est une polyoléfine et de préférence un polypropylène constitué en majeure partie de macromolécules isotactiques.

13. Une composition selon la revendication 12, caractérisée en ce que le dérivé d'alkyl-4-méthyl-pipéridine de formule générale (I) y est contenu en quantité comprise entre 0,01 et 2% en poids.

14. Une composition selon la revendication 10, caractérisée en ce que la substance polymère synthétique est un polymère vinylique halogéné.

15. Une composition selon la revendication 14, caractérisée en ce que le dérivé d'alkyl-4-méthyl-pipéridine de formule (I) est contenu dans celle-ci en quantité comprise entre 0,01 et 1% en poids par rapport au polymère vinylique halogéné.

16. Une composition selon la revendication 10, caractérisée en ce que la substance polymère synthétique est un polyuréthane, un polyamide ou un polymère dérivé d'un acide alpha, béta insaturé.

17. Une composition selon la revendication 16, caractérisée en ce que le dérivé d'alkyl-4-méthyl-pipéridine de formule générale (I) est contenu dans celle-ci en quantité comprise entre 0,01 et 5% en poids par rapport au poids de la substance polymère.

18. Une composition selon l'une quelconque des revendications 10 à 17, caractérisée en ce que le dérivé d'alkyl-4-méthyl-pipéridine de formule générale I est employé en mélange avec d'autres additifs choisis parmi des anti-oxydants, agents d'absorption de rayons UV, pigments, charges, polycondensats contenant de l'azote basique et agents de stabilisation.

19. Une composition selon la revendication 18, caractérisée en ce que les autres additifs sont employés, en même temps que le dérivé d'alkyl-4-méthyl-pipéridine de fomule générale (I) dans un rapport pondérale compris entre 0,5/1 et 3/1.